(19) Europäisches Patentamt
European Patent Office
Office européen des brevets

(11) EP 4 502 606 A1

(12) EUROPEAN PATENT APPLICATION

(43) Date of publication:
05.02.2025 Bulletin 2025/06

(21) Application number: 23306315.5

(22) Date of filing: 31.07.2023

(51) International Patent Classification (IPC):
G01N 33/68 (2006.01)   G01N 33/72 (2006.01)
G16H 50/20 (2018.01)

(52) Cooperative Patent Classification (CPC):
G01N 33/6893; G01N 33/6863; G01N 33/721;
G16H 50/20; G01N 2800/22; G01N 2800/60

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicant: Assistance Publique - Hôpitaux de
Paris
75012 Paris (FR)

(72) Inventor: The designation of the inventor has not
yet been filed

(74) Representative: Flesselles, Bruno F.G.
BF IP
36 rue Jean de la Fontaine
75016 Paris (FR)

(54) METHOD FOR DETERMINING HLH-ASSOCIATED DISEASE

(57) The invention pertains to a method to identify patients with an associated hemopathy within patients with Hemophagocytic Lymphohistiocytosis (HLH), using a combination of specific markers associated with bio-markers of the patient.

**Figure 1**

**Description**

The invention pertains to a method to identify patients with an associated hemopathy within patients with Hemophagocytic Lymphohistiocytosis (HLH), using a combination of specific markers associated with biomarkers of the patient.

Hemophagocytic syndrome (Hemophagocytic Lymphohistiocytosis, HLH) results in a loss of lymphocyte homeostasis and uncontrolled hyperactivation of the immune system, caused by uncontrolled proliferation of activated lymphocytes and macrophages. It is a life-threatening disease, as it can lead to organ failure due to the inflammatory process.

In children, HLH is a genetic disease and more than eleven genes (HLH- related genes) are currently identified which defects cause HLH. Moreover, HLH-related genes are unavoidable for the homeostasis of the granule dependent-cytotoxic function of CD8+ or NK cell.

Diagnosis of HLH can be performed with the HLH-2004 test, based on eight criteria (fever, splenomegaly, bicytopenia, hypertriglyceridemia and/or hypofibrinogenemia, and hemophagocytosis, low/absent NK-cell-activity, hyperferritinemia, and high-soluble interleukin-2-receptor levels). Five of these eight criteria must be fulfilled, unless family history or genetic diagnosis is consistent with HLH (Henter et al, Pediatr Blood Cancer. 2007 Feb;48(2):124-31. doi: 10.1002/pbc.21039). Fardet et al (Arthritis Rheumatol. 2014 Sep;66(9):2613-20. doi: 10.1002/art.38690) described the HScore, which uses nine variables (3 clinical [i.e., known underlying immunosuppression, high temperature, organomegaly], 5 biologic [i.e., triglyceride, ferritin, serum glutamic oxaloacetic transaminase, and fibrinogen levels, cytopenia], and 1 cytologic [i.e., hemophagocytosis features on bone marrow aspirate]), to obtain a score that can be used to estimate an individual's risk of having reactive hemophagocytic syndrome.

In adults, it occurs in association with two classes of pathologies: Hematological neoplasm-associated hemophagocytic lymphohistiocytosis (H-HLH) (hemopathies, in particular blood neoplasia (H-HLH)), or auto inflammatory or autoimmune diseases (AHAI)), or is considered idiopathic in the absence of the two preceding conditions (I-HLH). It is however to be noted that some infections (viral, in particular with EBV (Epstein Barr virus), HIV, human herpes virus or Cytomegalovirus, bacterial, fungal or parasitic) may also be present in patients HLH as booster of the syndrome whatever is the associated disease.

H-HLH is the most serious disease associated with HLH, with a mortality rate of over 50%, notably as the haemopathies are often very aggressive, which is a cause for this high mortality rate. B or T/NK cells-Non Hodgkin Lymphoma (NHL) or Hodgkin Lymphoma (HL) are most common causes of H-HLH, but it can also be associated with myeloproliferative syndrome.

Wang et al (Oncotarget. 2017 Jul 14;8(35):59977-59985. doi: 10.18632/oncotarget.19230) have reported multiple cancers that can be associated with HLH. Blood cancers are the most common (T-cell or NK-cell lymphoma, B-cell lymphoma, Hodgkin lymphoma, Leukemia, Not-specified hematologic neoplasms amounting to about 93.6% of the cancers associated with HLH, while solid tumors and non-specified neoplasms amount to about 6.4% of the other cancers).

- T-cell or NK-cell lymphoma: unspecified peripheral T-cell lymphoma, NK/T cell lymphoma, aggressive NK cell leukemia, gastric T-cell lymphoma, Anaplastic large cell lymphoma ALK negative, Anaplastic large cell lymphoma ALK positive, Angioimmunoblastic T-cell lymphoma, Panniculitis-like T-cell lymphoma, Gamma-delta ($\gamma\delta$) T-cell lymphoma, Lymphoblastic lymphoma/leukemia, Hepatosplenic T-cell lymphoma
- B-cell lymphoma: Diffuse large B-cell lymphoma, T-cell/histiocyte-rich large B-cell lymphoma, Follicular lymphoma, marginal zone lymphoma, intravascular large B-cell lymphoma, Burkitt's lymphoma
- Hodgkin lymphoma: Lymphocyte-depleted Hodgkin lymphoma
- Not specified lymphoma: Primary bilateral adrenal lymphoma
- Leukemia: Acute lymphoblastic leukemia (B-cell precursor acute lymphoblastic leukemia, T-cell acute lymphoblastic leukemia), Acute myelocytic leukemia, Acute monoblastic leukemia, Acute erythroid leukemia, Acute megakaryocytic leukemia, Chronic lymphocytic leukemia
- Other hematological neoplasms: Castleman's disease; Myelodysplastic syndromes , Multiple myeloma
- Solid tumor: Wilms tumor, Germ cell tumor, Lung cancer, prostate cancer, hepatocellular carcinoma, Colonic malignancy, Squamous cell carcinoma of the neck
- Not specified neoplasm: Mediastinal endodermal sinus tumor.

Early diagnosis of the pathology associated with HLH is a major challenge for clinicians, but can be made tricky by the difficulty of obtaining samples and their uncertain aspects in these fragile patients. Delay in diagnosing the associated pathology also leads to a delay in appropriate treatment, which compromises patient survival.

The usual action of physicians would first be to reach stabilization of the patient and organ support, then actively search for associated disease (malignancy, autoimmune or auto-inflammatory disease, or infection) to manage it.

Suppression of the overactive inflammatory response will also be sought.

Etoposide and corticosteroids or even cyclosporine A (this compound being less used due to side effects) are generally used for severely ill patients without associated hemopathy (neoplasia). In less severe disease, physicians generally would observe development of the disease without treatment, even though it is possible to start a treatment with steroids and/or intravenous immunoglobulins. Some antiviral molecules (such as rituximab) can also be used, in particular when EBV is present. On can also use anti-CD52 antibody alemtuzumab, JAK2 inhibitor ruxolitinib or another JAK1 or JAK2 inhibitor, anti-IFN-γ antibody emapalumab, plasma exchange, or cytokine adsorption using filter columns. If the patient's state starts to worsen, etoposide would be used.

Indeed, when neoplasia is detected in a HLH patient, it is possible to start an aggressive chemotherapy (cyclophosphamide, doxorubicin, vincristine, etoposide or corticosteroids such as prednisone or methylprednisolone). Etoposide can thus be added to CHOP (cyclophosphamide, doxorubicin, vincristine, and prednisone), CHOP-like. DEP (doxorubicin, etoposide, methylprednisolone) therapies are also used. Etoposide and corticosteroid drugs are thus added to chemotherapeutic drugs, and have an effect on both the cancer, and the uncontrolled inflammation due to HLH. However, such drugs can't be provided to all HLH patients, in particular due to side effects that can be associated with etoposide. Autologous or allogeneic stem cell transplantation can also be considered for these patients.

There is thus a need to have an early and easy test to identify the patients with H-HLH or not with H-HLH to be able to start and adjust an appropriate treatment (for instance start early administration of etoposide in H-HLH patients, or use other molecules for non H-HLH patients) as early as possible, and preferably before the results if deeper analysis (such as cytology to identify neoplasia) are available.

The inventors have shown that performing a combination of biologic markers and of the age of the patient makes it possible to quickly determine whether the patient has a neoplasia (an hemopathy associated with the HLH syndrome), or not. This makes it possible to transfer the patient to a specialized center and start an appropriate treatment as early as possible, for some patients that are at risk of worsening and of death.

Zhou et al (J Clin Immunol. 2022 Jul;42(5):1000-1008. doi: 10.1007/s10875-022-01263-z) developed various models using biomarkers (age, ferritin, lymphocyte (LY), international normalized ratio (INR), thrombin time (TT), globulin, uric acid (UA), chloride, activated partial thromboplastin time (APTT), aspartate aminotransferase (AST), triglycerides (TG), total bilirubin (TB), and indirect bilirubin (IB)) to predict 30-day death in total and subgroup HLH patients.

Shiga et al (Front Immunol. 2021 Oct 8;12:750114. doi: 10.3389/fimmu.2021.750114) showed that IL-18 could be a useful biomarker for the differential diagnosis of Adult-Onset Still's Disease and Hemophagocytic Lymphohistiocytosis in adults.

Gao et al (Front Immunol. 2023 Apr 28;14:1143181. doi: 10.3389/fimmu.2023.1143181) established a predictive model to help clinicians diagnose the original disease resulting in secondary HLH during routine practice, using a retrospective study of 175 secondary HLH patients in this study, including 92 patients with hematologic disease and 83 patients with rheumatic disease. The authors showed that lower levels of hemoglobin and platelets (PLT), lower levels of ferritin, splenomegaly and Epstein-Barr virus (EBV) positivity were associated with hematologic disease, but young age and female sex were associated with rheumatic disease. The risk score included assessments of female sex, age, PLT count, ferritin level and EBV negativity to predict HLH secondary to rheumatic diseases.

In diagnosis tests, it is reminded that

(1)Sensitivity is the probability that the diagnosis is positive in individuals having the phenotype sought (detection of true positives): the test is positive if the patient is having the phenotype. The sensitivity is low when the number of false negatives is high. The sensitivity is calculated by the formula SE = (number of individuals having the phenotype in whom the sign is present)/(number of individuals having the phenotype in whom the sign is present + number of individuals having the phenotype in whom the sign is absent).

(2)Specificity is the probability that the diagnosis is negative in the individuals not having the phenotype sought (non-detection of true negatives): the test is negative if the patient is not suffering from the disease. The specificity is low when the number of false positives is high. The specificity is calculated by the formula SP = (number of individuals not having the phenotype in whom the sign is absent)/(number of individuals not having the phenotype in whom the sign is absent + number of individuals not having the phenotype in whom the sign is present).

(3)Positive predictive value (PPV): is the probability of having the disease if the diagnostic test is positive (i.e. that the patient is not a false positive): the patient is having the phenotype if the test is positive. The positive predictive value is calculated by the formula PPV = (number of individuals having the phenotype in whom the sign is present)/(number of individuals having the phenotype in whom the sign is present + number of individuals not having the phenotype in whom the sign is present).

(4)Negative predictive value (NPV): is the probability of not having the disease if the diagnostic test is negative (that the patient is not a false negative): the patient is not having the phenotype if the test is negative. The negative predictive value is calculated by the formula NPV = (number of individuals not having the phenotype in whom the sign is absent)/(number of individuals not having the phenotype in whom the sign is absent + number of individuals having the phenotype in whom the sign is absent)

In order to obtain a good diagnostic test, it is important to both increase specificity and sensitivity.

The quality of a diagnostic test is generally determined by drawing a Receiving Operating Characteristic (ROC) curve and measuring the Area Under Receiving Operating Characteristic curve (AUROC).

The ROC curve is drawn by plotting the sensitivity versus (1-specificity), after classification of the patients, according to the result obtained for the test, for different thresholds (from 0 to 1).

It is usually acknowledged that a ROC curve, the area under which has a value superior to 0.7, is a good predictive curve. The ROC curve has to be understood as a curve allowing prediction of the quality of a classification test. It is best for the AUROC to be as closed as 1 as possible, this value describing a test which is 100 % specific and sensitive. Consequently, a test with a higher AUROC than another is a better classifier than the other.

Generally, a diagnosis method comprises

i. a step of gathering information from the patient
ii. a step of comparing said information with regards to thresholds
iii. a step of deducing, from the difference between the patient's information and the threshold, whether the patient has a specific disease, the stage of the patient's disease, or whether the patient's state will evolve to a given state.

As a matter of illustration

i. the information that can be gathered from the patient can be gathered directly from the patient (such as images from NMR, scanner, radiography, contrast-enhanced computed tomography), or indirectly from the patient, such as from a biological sample that has been obtained from a patient (such as urine, blood sample...). The information can be presence (or absence) and/or level of specific biological markers, whether specific from the pathogenic determinant (bacterial or viral DNA/RNA), or elevated levels of patient's markers
ii. once the information is obtained, it is compared to different values / standards and the deviation with regards to these standards is assessed. As a matter of illustration, the level of some biomarkers shall be compared to the level usually observed in healthy patients and to the levels usually observed in patients with the disease (or for patients who have been known to later evolve to a specific disease stage, for prognosis methods). Thresholds may exist, where 95 % of patients having passed the threshold have the disease and 95 % of the patients not having passed the threshold do not have the disease. For diseases where multiple clinical stages can be determined, such thresholds can discriminate the different stages. In this step ii, one may compare various types of information to their respective standards, in order to be able to reach a diagnostic in step iii (as a matter of illustration, one can use the values and information obtained from measurement of various blood or plasma markers, images from scanner and of Body Mass Index).
iii. the last step is actually making the diagnosis (resp. determining a prognosis) *i.e.* deciding whether or not the patient has the condition sought resp. whether or not the patient will evolve to a given clinical state), taking, in particular, into account the information gathered from the patient, the thresholds as described above. The physician may also take into account other elements (such as the consistency of the information gathered or the like) to make the diagnostic.

Some methods, such as the ones disclosed in the present application, shall also include a step i.a), which comprise the steps of modifying the information obtained from the patient in order to obtain a new type of information, which is the one that is then compared to the standards in step ii. Such modification is the combination of the values of variables in a function, and obtaining an end value.

It is further to be noted that the mere measurement of the values of levels of markers in the plasma or serum of a patient and the combination thereof in an algorithm or function as herein disclosed is part of a method but only provides an intermediate result (an end value or index) that would then to be compared to a reference index (threshold), in order to be able to pose the diagnostic.

It is also to be noted that the tests herein disclosed are not "gold-standard" tests, in the sense that the output (end value or index calculated by the formulas herein disclosed) is not a definitive answer as to the state of the patient. Indeed, these tests are based on statistics and there may thus be false-positive or false-negative results, which is the reason why the specific experience of the physician in interpreting the index is of importance for making the prognosis and deciding which kind of follow up is to be made to ne made for each patient.

However, due to the specificity, sensitivity, positive predictive value and negative predictive value of the tests, herein provided for various thresholds of the index, these tests are of great interest in provided a help to the physician when investigating a clinical case. Consequently, step iii as disclosed above is not direct and immediate from step ii, as the physician must interpret the result from the clinical and general context to be able to reach a conclusion.

The invention thus relates, in a first aspect, to a method for determining whether an hemopathy is associated with HLH in an adult subject (or whether the adult subject has an increased risk of having an hemopathy (blood neoplasy) associated with HLH (H-HLH)), said subject having thus been diagnosed with HLH, comprising

a) Obtaining the age in years of the subject
b) Obtaining the values at least three biomarkers measured in the blood, plasma or serum of the subject, wherein the biomarkers are selected in the group consisting of hemoglobin, platelets, interleukin-1 beta(IL-1b), interleukin 6 (IL-6), interleukin 10 (IL-10), Tumor Necrosis Factor $\alpha$ (TNF-$\alpha$), interleukin 18 (IL-18), interferon gamma-induced protein 10 (IP-10), soluble Interleukin-2 receptor alpha chain (sIL2-R-$\alpha$, CD25) and CD4+/CD8+ ratio,
c) optionally obtaining values of other biomarkers
d) Combining values associated with the values obtained in a), b) and c) in a function, and obtaining an output
e) comparing the output to a predetermined cutoff value, and determining presence of an associated with HLH if the output is higher than the predetermined cutoff value.

This method is performed *ex vivo.* It is preferably computer-implemented. In particular, step d) is computer-implemented.

In b) the values obtained for the biological markers are the amount (measured in the units that are classically used when assessment of the level of these markers is performed at the hospital or in biological laboratories.

Preferably, the at least three biomarkers are selected in the group consisting of hemoglobin, platelets, interleukin-1 beta(IL-1b), interleukin 6 (IL-6), interleukin 10 (IL-10), Tumor Necrosis Factor $\alpha$ (TNF-$\alpha$), interleukin 18 (IL-18), interferon gamma-induced protein 10 (IP-10), soluble Interleukin-2 receptor alpha chain (sIL2-R-$\alpha$, CD25) and CD4+/CD8+ ratio.

In another embodiment the at least three biomarkers comprise interleukin 10 (IL-10), Tumor Necrosis Factor $\alpha$ (TNF-$\alpha$), interleukin 18 (IL-18) or CD4+/CD8+ ratio.

Step c) is optional and the combination of d) can thus be performed with only the age of the patient and the values of the at least three markers listed in b), preferably chosen from interleukin 10 (IL-10), Tumor Necrosis Factor $\alpha$ (TNF-$\alpha$), interleukin 18 (IL-18) and CD4+/CD8+ ratio.

In the context of the present application, the risk is increased as compared with the risk of the patients not for which the output is below the predetermined cutoff value. As an illustration, if the risk of presence of an hemopathy associated with HLH in the population of patients with an output lower than the predetermined cutoff value is x %, the risk of presence of an hemopathy associated with HLH in the population of patients with an output higher than the predetermined cutoff value would be higher than x %. A risk of x % within a population indicates that, in the population, x % of the patients present the required phenotype (here presence of an hemopathy associated with the HLH syndrome). In some embodiments, the risk of having H-HLH increase by a factor of 12 for patients with an output higher or equal than the predetermined cutoff value compared to patients with an output below the predetermined cutoff value.

Other circulating biomarkers can be used in the method according to the invention. In particular, it is advisable to use at least one marker selected from the amounts of ferritin, lymphocyte (LY) globulin, uric acid (UA), chloride, aspartate aminotransferase (AST), triglycerides (TG), total bilirubin (TB), and indirect bilirubin (IB), and the international normalized ratio (INR, clotting time), the thrombin clotting time (TT), and the activated partial thromboplastin clotting time (APTT).

It is also possible to use amounts of ferritin, presence of splenomegaly and of the Epstein-Barr virus (EBV), in particular amount of ferritin, and presence of the Epstein-Barr virus (EBV), as well as the sex of the patient, as other markers used in c).

In order to perform the method, one can use, in d), the values that are directly measured or obtained from the patients, in a), b) or c).

In another embodiment, it is possible to take the information measured or obtained from a), b) or c) and to use a value associated with it. In particular, it is possible to define predetermined thresholds specific for each marker, and to assign the value 1 of the amount of the marker is above its predetermined threshold and the value 0 if the amount of the marker is below such predetermined threshold.

Such 0 and 1 values thus obtained will then be used in the function of d), as the values associated with the values obtained in a), b) and c).

The method thus can be performed with the value associated with each marker obtained by

a. comparing the values obtained in a), b) and c) to predetermined thresholds specific for each marker, and
b. assigning the value 1 if the value is above the predetermined threshold for the marker, or the value 0 if the value is below or equal the predetermined threshold.

The predetermined threshold for each marker can be determined by the person skilled in the art. For each marker, one can, for instance, determine the predetermined threshold as being the median values observed for this marker in for patients with HLH.

In one embodiment, the predetermined thresholds are as depicted in Table 1.

Table 1. Predetermined thresholds for biomarkers

| Variable | Threshold |
|---|---|
| Age | 48 years |
| Hemoglobin | 9 g/l |
| Platelets | 82 G/l |
| IL-1$\beta$ | 6 pg/mL |
| IL-6 | 46 pg/mL |
| IL-10 | 42 pg/mL |
| TNF-$\alpha$ | 43 pg/mL |
| IL-18 | 574 pg/mL |
| IP-10 | 10004 pg/mL |
| sIL2-R-$\alpha$ | 37 pg/mL |
| CD4/CD8 ratio | 1 |

In the preferred embodiment, the function is a logistic function, *i.e.* a function that has been obtained after performing a logistic regression, using the markers obtained or measured in a), b) and c) as the independent variables that are combined in a linear function (the logistic function), the coefficients being for instance obtained by the maximum-likelihood estimation (MLE).

In particular, the function can be obtained by

a. determining the presence of an hemopathy in a cohort of patients with HLH, wherein the values of circulating biochemical markers are known for the patients, and wherein the age is also known
b. identifying by univariate logistic regression analysis, the circulating biochemical markers for which the values differ significantly between the group of patients with an hemopathy and the group of patients without presence of an hemopathy
c. performing a multivariate logistic regression analysis to assess and ponder the independent discriminative value identified in step b) for the occurrence of hemopathy,
d. thereby obtaining a function by combination of these identified independent factors.

The coefficients of the function thus ponder the weights of each independent factor when used in the combination.

The inventors have shown that age, hemoglobin, platelets, interleukin 10 (IL-10), Tumor Necrosis Factor $\alpha$ (TNF-$\alpha$), interleukin 18 (IL-18), soluble Interleukin-2 receptor alpha chain (sIL2-R-$\alpha$, CD25), and CD4+/CD8+ ratio are markers that differ significantly in univariate analysis between the group of patients with an hemopathy and the group of patients without presence of an hemopathy (p<0.05) and some of such markers can thus be selected for the multivariate logistic regression analysis of c. When other markers are added (such as amount of ferritin or presence of the EBV virus), they can also be included in the regression analysis. One can note that the quality of the obtained function (and thus of the test) can be determined by the Area under the ROC curve, as indicated above, and that addition or deletion of such markers can be performed without modifying the quality. Generally, it is preferred to use the markers that can be readily obtained, and to use the fewer number of markers possible, which provide the highest sensitivity or specificity (as this also allows to keep test costs low).

In one specific embodiment, at least four biomarkers are selected in b).

In one embodiment, the at least four biomarkers comprise interleukin 10 (IL-10), Tumor Necrosis Factor $\alpha$ (TNF-$\alpha$), interleukin 18 (IL-18) and CD4+/CD8+ ratio. In particular four biomarkers are selected in b) and are IL-10, TNF-$\alpha$, IL-18 and CD4/CD8 ratio. Their value is then combined with the age of the patient to obtain the end result that provides information as to the presence of the hemopathy associated with HLH.

When the biomarkers selected in b) are IL-10, TNF-$\alpha$, IL-18 and CD4/CD8 ratio, the function is preferably

a1 x Age [0 if ≤48 years; 1 if > 48 years] + a2 x IL-10 [0 if ≤42; 1 if >42]

- a3 x TNF-$\alpha$ [0 if ≤ 43; 1 if > 43] + a4 x IL-18 [0 if ≤ 574; 1 if > 574] – a5

x CD4/CD8 ratio [0 if ≤ 1; 1 if > 1]

with

$$10 \leq a1 \leq 12$$

$$14 \leq a1 \leq 16$$

$$15 \leq a3 \leq 17$$

$$10 \leq a4 \leq 12$$

$$11 \leq a5 \leq 13$$

This function thus uses values associated with the measured or obtained value of the variable of the patient, determined as indicated above. Thus, if the patient's age is higher than 48 years, the value "1" is used in the function and the value "0" is used if the age of the patient is lower or equal than 48 years.

In a preferred embodiment, the function is:

$$11 \times \text{Age [0 if} \leq 48 \text{ years; 1 if} > 48 \text{ years]} + 15 \times \text{IL-10 [0 if} \leq 42; 1 \text{ if} > 42]$$
$$- 16 \times \text{TNF-}\alpha \text{ [0 if} \leq 43; 1 \text{ if} > 43] + 11 \times \text{IL-18 [0 if} \leq 574; 1 \text{ if} > 574] - 12$$
$$\times \text{CD4/CD8 ratio [0 if} \leq 1; 1 \text{ if} > 1].$$

Such function thus will give discrete outputs comprised between -28 and 37 (limits included). A value of 6 as the predetermined cutoff (patients with an output equal or higher than 6 have a blood neoplasy associated with HLH) provides a sensitivity of 0.8 and a specificity of 0.75. The PPV is 0.68 and the NPV is 0.85.

It is also possible to normalize the outputs to have them comprised between 0 and 1. For instance, it is possible to use the function

$1.1 \times$ Age [0 if $\leq$48 years; 1 if > 48 years] + $1.5 \times$ IL-10 [0 if $\leq$42; 1 if >42] - $1.6 \times$ TNF-$\alpha$ [0 if $\leq$ 43; 1 if > 43] + $1.1 \times$ IL-18 [0 if $\leq$ 574; 1 if > 574] - $1.2 \times$ CD4/CD8 ratio [0 if $\leq$ 1; 1 if > 1] to obtain an intermediate result $r$, and to obtain the output $o$ as $o$=1-1/(1+exp($r$)). When such normalization is performed, an output $o$ equal or higher than 0.646 indicates a patient with a higher risk of blood neoplasy associated with HLH. It is also possible to use output $o1$ = ox0.500/0.646, to determine a cutoff of 0.5 as the cutoff to identify the patients with higher risk of neoplasy.

As indicated above, the method is to be performed on adult subjects (as HLH in children essentially results of genetic causes), which have been diagnosed as having HLH. It is preferred that such diagnosis has been obtained by the subject being positive to the H-score or the HLH-2004 test, as described above.

The method is preferably implemented, in totality or partially by a computer. In particular, step d) is computer-implemented. When a dichotomous (or binary) value (0/1) is used in the function, it is preferred when the step of comparing the value obtained or measured for a marker to the predetermined threshold specific for the marker and transforming the value to "0" or to "1" is performed with the use of a computer.

In some embodiments, the invention relates to a computer-implemented method for diagnosing the presence of an hemopathy associated with HLH in a subject (or increased risk of H-HLH), wherein the subject has HLH comprising

a) Receiving inputs from a sender, wherein the inputs are the values of biomarkers measured in the blood, serum or plasma of the subject, as well as the information pertaining to the age of the subject, and optionally to the (genetic) sex of the subject

b) Performing a method as described above, to obtain an output for the combination of the values received in (a)

c) optionally comparing the output to a reference value and determining the presence of the hemopathy associated with HLH (or the increased risk of H-HLH)

d) Sending the output to the sender and/or the presence of the hemopathy associated with HLH (or the increased risk of H-HLH), if determined

e) Optionally storing the inputs and the output in a database.

The genetic sex is male for XY chromosomes and female for XX chromosomes.

In some embodiments, the invention relates to a computer implemented method for obtaining information as to the presence of the hemopathy associated with HLH in a subject (or the increased risk of H-HLH), wherein the subject has HLH comprising

a) Sending inputs to a server, wherein the inputs are the values of the concentration of at least three measured biological markers selected from the group consisting of hemoglobin, platelets, interleukin-1 beta(IL-1$\beta$), interleukin 6 (IL-6), interleukin 10 (IL-10), Tumor Necrosis Factor $\alpha$ (TNF-$\alpha$), interleukin 18 (IL-18), interferon gamma-induced protein 10 (IP-10), soluble Interleukin-2 receptor alpha chain (sIL2-R-$\alpha$ , CD25), and CD4+/CD8+ ratio, and the age of the subject

b) Having the remote server combine the values received in (a) in a function, described above, and obtain an output [end result], optionally normalized

c) optionally having the remote server compare the output to a reference value and determining the presence or absence of the hemopathy associated with HLH in the subject (and/or the increased risk of H-HLH)

d) Optionally having the remote server store the inputs and the output in a database

e) receiving the output from the server and/or the information pertaining to the presence or absence of the hemopathy associated with HLH and/or the increased risk of H-HLH (when determined).

In other embodiments, the invention relates to a computer-implemented method for determining whether a patient with HLH has an hemopathy associated with HLH (or an increased risk of H-HLH)

a) requiring a sender to identify himself to a server within a public or private network,

b) requiring the sender to fill in information related to the patient, and assigning a specific identifier to the patient in a database, (this last step may be omitted if the patient has already been recorded in the database, as can be determined, using the patient information);

c) receiving values of the concentration of at least three measured biological markers selected from the group consisting of hemoglobin, platelets, interleukin 1-beta (IL-1$\beta$), interleukin 6 (IL-6), interleukin 10 (IL-10), Tumor Necrosis Factor $\alpha$ (TNF-$\alpha$), interleukin 18 (IL-18), ), interferon gamma-induced protein 10 (IP-10), soluble Interleukin-2 receptor alpha chain (sIL2-R-$\alpha$ , CD25), and CD4+/CD8+ ratio, and the age (it may be calculated from the birth date), wherein the values are received in a secure manner, and storing the values in the database, associated with the specific identifier of the patient

d) combining the values received or calculated in c) by use of a function described above, obtaining a result, and optionally normalizing the result

e) storing the result optionally normalized of d) in a database associated with the specific identifier and preferably the date and time of identification of the sender, thereby obtaining a database where the information related to the patient is present under the specific identifier,

f) sending the nature of the presence of the hemopathy associated with HLH to the sender (or of the increased risk of H-HLH), wherein the patient has the hemopathy associated with HLH if the result ((or an increased risk of H-HLH) is above or equal a predetermined value and the patient has not the hemopathy associated with HLH (or no increased risk of H-HLH) if the result is below the or another predetermined value.

In particular, to implement the above methods, a sender is required to identify himself to a server or system within a public or private network. It is preferred when the network is a private network, and when the connection to the server is encrypted. Identification of the sender can be performed using login and password, preferably through strong identification processes (such as one-time password, or using biometric or smart card identification).

Upon login to the server, the sender is required to fill-in information related to the patient (such as name, sex, birth date, height, weight, social security identification number, social insurance number) and a specific identifier is assigned to the patient in a database. If the database does not contain any entry relating to the patient, one entry is created. If an entry already exists (using the social security identification number allows ensuring the unicity of entry), such entry is activated.

The server shall then receive values of the concentration of biochemical markers as disclosed herein, in particular at least three markers (or at least four markers) chosen from hemoglobin, platelets, interleukin-1 beta (IL-1$\beta$), interleukin 6 (IL-6), interleukin 10 (IL-10), Tumor Necrosis Factor $\alpha$ (TNF-$\alpha$), interleukin 18 (IL-18), interferon gamma-induced protein 10 (IP-10), soluble Interleukin-2 receptor alpha chain (sIL2-R-$\alpha$, CD25) and CD4+/CD8+ ratio, and store them in a database, associated with the specific identifier of the patient, so that such data is uniquely associated to the patient. Exchanges between the sender's device and the server are very advantageously performed in a secure manner (encrypted exchanges). Age of the patient is to be sent or may be calculated using the information related to the patient, as is sex if required. Such information is also preferably received in a secure manner, and stored in the database, associated with the specific identifier of the patient.

In one embodiment the at least three or four markers are chosen from hemoglobin, platelets, interleukin-1 beta

(IL-1β), interleukin 6 (IL-6), interleukin 10 (IL-10), Tumor Necrosis Factor α (TNF-α), interleukin 18 (IL-18), interferon gamma-induced protein 10 (IP-10), soluble Interleukin-2 receptor alpha chain (sIL2-R-α, CD25), and CD4+/CD8+ ratio.

In one embodiment, the at least three or four markers comprise at least interleukin 10 (IL-10), Tumor Necrosis Factor α (TNF-α), interleukin 18 (IL-18) and CD4+/CD8+ ratio. In one embodiment, four markers are chosen and are interleukin 10 (IL-10), Tumor Necrosis Factor α (TNF-α), interleukin 18 (IL-18) and CD4+/CD8+ ratio.

It is to be noted that the sender may be in a remote place different from where the server is located. It is also envisaged that the information is recovered by the server, using information provided by the sender. For instance, the server may open a connection with the server of the laboratory having measured the biochemical markers, using identifiers provided by the sender, to upload the required information.

A next, optional, step would be to obtain values associated with the markers as indicated above (binary 0/1 values depending on the comparison of the obtained or measures value of the markers with thresholds specific for each used marker).

The next step is to combine the values of the concentration of the markers, or the values associates with the markers in a function as disclosed herein to obtain a result, that is optionally normalized as disclosed.

The information pertaining to the comparison is then stored in a database associated with the identifier specific of the patient and is preferably date and time-stamped. Indeed, since the method is to be performed on a regular basis, it may be interested to keep a record of the results obtained overtime. Thereby, a database is obtained, containing identification related to the patient (patient's specific identifier), associated with the biochemical data, and comparison information.

The nature of the risk of the presence of H-HLH for the patient (presence of absence) is thus determined according to the methods herein disclosed.

Such information (nature of the risk of the patient) is then transmitted to the sender, optionally with the result of the function and with any other potential information of interest.

The invention thus makes it possible to provide appropriate care and treatment for patients according to their risk of having H-HLH.

In particular, one can implement a method for treating a patient, comprising

a) Performing methods as described above, to determine presence of H-HLH or increased risk of H-HLH,
b) Transferring the patient to clinical unit specialized in hematologic disorders, and specially in hematologic cancers and/or providing an adapted chemotherapy and/or performing supplementary analysis to confirm the nature of the hemopathy associated with HLH, when the output indicates presence of an hemopathy associated with HLH for the patient, or an increased risk of H-HLH.

The present invention provides a tool for the clinician to be able to identify patients that have an increased risk of having H-HLH. In view of the HLH symptoms, patients with HLH are generally first seen in internal medicine or infectious diseases departments. Before sending the patient to cancer specialized services to characterize a malicious disease associated with HLH, the physicians will first try to find the underlying cause of fever and inflammation see in the patient, and this can lead to a loss of time in diagnosing the associated hemopathy and patient management with appropriate medication. Consequently, the present invention provides a direct and real impact on the patient care, in that it will lead to an early transfer of the patient to specialized units for future characterization (and potentially early treatment), thus improving the patient's chances. It is also possible to choose the specific predetermined cutoff value to increase the PPV (resp the NPV) to increase the likelihood that a patient with an end value higher than such predetermined cutoff value has a H-HLH (resp. that a patient with an end value below than such predetermined cutoff value has not a H-HLH).

In particular, chemotherapy comprises administration of etoposide and/or of corticoids. As indicated above, one can apply a CHOP, CHOP-like treatment to the patient supplemented with etoposide, or a DEP treatment. The goal of the treatment is to start fighting the blood neoplasia as soon as possible (even before the type of cancer has been fully determined) and avoid quick worsening of the state of the patient.

The supplementary analysis may comprise Bone Marrow Biopsy, PET-Scan analysis, liver biopsy, splenectomy. It is also possible to look for circulating markers that are specific of blood cancers, or to perform histology of the circulating cells of the subject. Serological markers of non-Hodgkin lymphomas include levels of Lactate dehydrogenase (LDH), Beta-2 microglobulin (β2-M), CA 125, Interleukin-6 (IL-6). Immunophenotypic markers include B or T lineage, maturation level / disease specific, proliferative activity (Ki-67), clonality (light chains of Ig). Molecular markers include detection of activation of proto-oncogenes (such as BCL-1, BCL-2, BCL-6, c-MYC), inactivation of tumor suppressor genes (such as p53)- infection of tumor clones with oncogenic viruses (such as EBV and HHV-8). For B lymphomas, one can also cite CD20 (in B-lymphomas, whereas it is absent in T-lymphomas), CD79a, Oct-2 and BOB.1. Hodgkin lymphoma express CD15, CD30, CD57. For T/NK-cell lymphomas, one can cite CD2, CD3, CD5, CD7, CD8, CD56 and CD57. One can also cite Bcl-1, Bcl-2, Bcl-6, CD10, Multiple myeloma oncogene (MUM1), CD138 (Syndecan-1), CD43, Anaplastic lymphoma kinase (ALK), Terminal deoxynucleotidyl transferase (TdT), Granzyme B, perforin and TIA-1, as markers that can be used to identity and characterize a hematologic cancer.

It is also possible to implement a method for treating a patient, comprising

a) Performing the method as described above to determine whether the patient has an increased risk of having H-HLH, and

b) Administering a JAK inhibitor to the patient when the output doesn't indicate presence of an hemopathy associated with HLH for the patient or an increased risk.

Keenan et al (Front Immunol. 2021 Feb 16;12:614704. doi: 10.3389/fimmu.2021.614704) have discussed the use of ruxolitinib (JAK2 inhibitor) in the treatment of HLH, and Das et al (Blood. 2016 Mar 31;127(13):1666-75. doi: 10.1182/blood-2015-12-684399) have also reported that Janus kinase inhibition lessens inflammation and ameliorates disease in murine models of hemophagocytic lymphohistiocytosis.

The invention also relates to a device for determining whether a HLH patient has an hemopathy associated with HLH or an increased risk of having H-HLH, comprising

a. a keyboard for entering information

b. software to present an information input interface

c. a processor to calculate the values associated with the information entered by the user

d. a processor for calculating the output result according to a method as disclosed

e. a monitor for displaying the output result, as well as the presence or absence of an hemopathy associated with HLH for the patient, or whether the patient has increased risk of HLH.

The device shall present a keyboard (physical or virtual keyboard) that makes it possible to enter information pertaining to the age of a patient, as well as to the values of the biological markers that have been measured from the blood, serum or plasma of the patient. The information are entered *via* an information input interface that prompts the user to enter the information, and preferably to validate the inputted information.

Such values can be stored in a database that is embodied in the device, such storage being preferably transient (the database being emptied after a certain period of time, or when the information has been sent to a permanent database, such sending being carried by any method of the art (such as Bluetooth, wifi, optical fiber... ).

A processor may be present to calculate the value associated with the value entered by the user, as indicated above. Such associated values can be stored in a transient database or transient memory.

A processor shall calculate, using a pre-stored function, the output (end value), compare the result with a predetermined cutoff value to determine whether the patient has an increased risk of H-HLH.

The device may also include a monitor to display the calculated output and whether the patient has or not an increased risk of H-HLH. Color codes (red in case of a risk) may be used to reinforce the provided information when the patient has an increased risk of H-HLH.

Is also foreseen a non-transitory computer readable storage medium, having stored thereon a computer program comprising program instructions, the computer program being loadable into a data-processing unit and adapted to cause the data-processing unit to carry out a method for calculating a first index by combining the values as measured from markers present in the serum or plasma of a patient through a function as disclosed above, when the computer program is run by the data-processing device.

In another embodiment, the invention relates to a microprocessor comprising a computer algorithm to perform a method of determining the presence of H-HLH (or an increased risk of H-HLH) in a patient: providing the values of blood markers of a patient and the age; and combining the values through a function (as disclosed above, or obtained as disclosed above) to obtain a score (the end-result or first index) useful for presence of H-HLH or the increased risk of H-HLH in the patient.

The invention also relates to the use of the functions, devices and/or microprocessors in order to make a diagnosis of presence of H-HLH or the increased risk of H-HLH in a patient by use of the functions and comparing the result to a predetermined threshold to determine the presence of H-HLH or the increased risk of H-HLH in the patient.

**Description of the Figures**

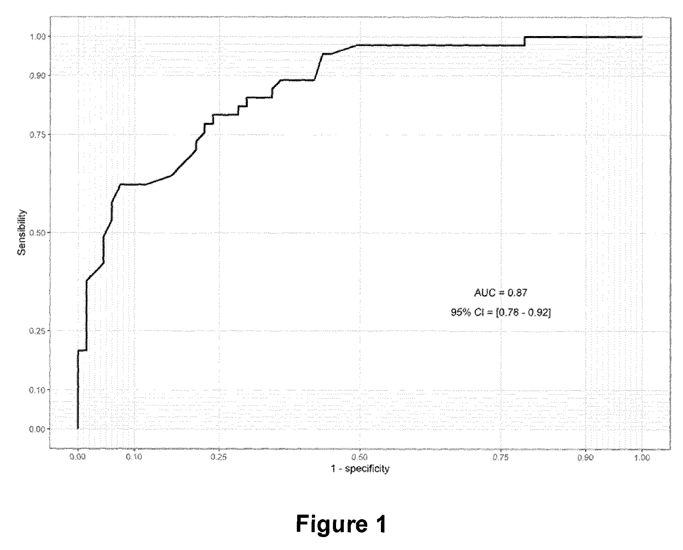

Figure 1: ROC curve of the diagnosis model described in the example

**Example**

Methods

Thirty cytokines serum level were analysed by Elisa or by Luminex Technology, in 112 patients (age≥18 years old and "HScore"≥169) of the French HLH cohort data base (clinicaltrials.gov number NCT02113917). Multiple imputation chain equation was performed to account for the missingness in covariates. All following analyses were carried out based on the imputed datasets by combining estimates from each completed dataset using Rubin's rules. A logistic regression was used to develop and validate the risk score predicting H-HLH. Model calibration and discrimination were assessed using the Hosmer-Lemeshow and receiver operating characteristic (ROC) curves, respectively. A 10-fold cross-validation was carried out to take into account overfitting bias. Risk scores were derived using the model parameters and individual covariates values. A threshold on the risk score was determined by maximizing the Youden index (sensitivity+specificity-1). H-HLH High-risk and low-risk Patients were then delineated according to this score threshold, respectively defining a high risk H-HLH group.

Description of the clinical cohort is provided in Table 2

**Table 2**. Cohort clinical data

| Variable | ALL N=112 | Other (autoimmune disease + idiopathic disease) N=67 | Neoplasia N=45 |
|---|---|---|---|
| **Median age (year) [IQR]** | 48 [36;61] | 41 [30;55] | 56 [44;68] |
| **Missing data** | 0 (0%) | 0 (0%) | 0 (0%) |
| **Male, N(%)** | 64 (57%) | 34 (51%) | 30 (67%) |
| **Missing data** | 0 (0%) | 0 (0%) | 0 (0%) |
| **Splenomegalia, N(%)** | 62 (100%) | 30 (100%) | 32 (100%) |
| **Missing data** | 50 (45%) | 37 (55%) | 13 (29%) |
| **Median Fibrinogen, g/l , [IQR]** | 3 [2;5] | 3 [2;4] | 4 [2;5] |
| **Missing data** | 29 (26%) | 18 (27%) | 11 (24%) |
| **Median total Lymphocytes, /mm^3, [IQR]** | 730 [396;1388] | 1068 [572;1744] | 600 [390;890] |
| **Missing data** | 41 (37%) | 25 (37%) | 16 (36%) |
| **Median Lymphocytes, /mm^3, [IQR]** | 715 [308;1024] | 770 [340;1100] | 700 [300;960] |
| **Missing data** | 20 (18%) | 14 (21%) | 6 (13%) |
| **Median ASAT, UI/l, [IQR]** | 138 [58;296] | 158 [62;332] | 83 [53;223] |
| **Missing data** | 8 (7%) | 4 (6%) | 4 (9%) |
| **Median ALAT, UI/l, [IQR]** | 81 [42;211] | 109 [54;280] | 57 [34;127] |
| **Missing data** | 7 (6%) | 4 (6%) | 3 (7%) |
| **Median Hemoglobin, g/l, [IQR]** | 9 [8;11] | 9 [8;11] | 8 [8;10] |
| **Missing data** | 1 (1%) | 0 (0%) | 1 (2%) |
| **Median platelets, G/l, [IQR]** | 82 [41;125] | 98 [64;142] | 66 [33;104] |
| **Missing data** | 3 (3%) | 3 (4%) | 0 (0%) |

(continued)

| Variable | ALL N=112 | Other (autoimmune disease + idiopathic disease) N=67 | Neoplasia N=45 |
|---|---|---|---|
| **Median Neutrophile, /mm$^3$, [IQR]** | 2130 [1201;4622] | 2700 [1500;4900] | 1620 [903;4210] |
| **Missing data** | 16 (14%) | 10 (15%) | 6 (13%) |
| **Median natraemia, mmol/l, [IQR]** | 132 [129;137] | 134 [129;138] | 132 [129;136] |
| **Missing data** | 14 (12%) | 10 (15%) | 4 (9%) |
| **Median Ferritin, μg/L, [IQR]** | 7000 [2144;14760] | 6288 [2143;10578] | 8482 [2300;18014] |
| **Missing data** | 7 (6%) | 7 (10%) | 0 (0%) |
| **Median TP,% , [IQR]** | 75 [61;87] | 75 [65;89] | 72 [60;81] |
| **Missing data** | 28 (25%) | 19 (28%) | 9 (20%) |
| **Median CD8/CD25+, c/mm$^3$, [IQR]** | 73 [58;161] | 65 [52;161] | 80 [62;119] |
| **Missing data** | 99 (88%) | 62 (93%) | 37 (82%) |
| **Infection, N(%)** | | | |
| **None** | 42 (38%) | 20 (30%) | 22 (50%) |
| **Herpes Virus** | 39 (35%) | 21 (31%) | 18 (41%) |
| **Intracellular bacteria** | 16 (14%) | 16 (24%) | 0 (0%) |
| **Herpes Virus and others** | 3 (3%) | 2 (3%) | 1 (2%) |
| **Others** | 11 (10%) | 8 (12%) | 3 (7%) |
| **Missing data** | 1 (1%) | 0 (0%) | 1 (2%) |
| **Severity, N(%)** | | | |
| **Benign** | 41 (37%) | 32 (48%) | 9 (20%) |
| **Serious** | 71 (63%) | 35 (52%) | 36 (80%) |
| **Missing data** | 0 (0%) | 0 (0%) | 0 (0%) |
| **Number of variants, N(%)** | | | |
| **0** | 55 (51%) | 36 (54%) | 19 (48%) |
| **1** | 36 (34%) | 19 (28%) | 17 (42%) |
| **> 1** | 16 (15%) | 12 (18%) | 4 (10%) |
| **Missing data** | 5 (4%) | 0 (0%) | 5 (11%) |
| **Median IL-1β, pg/mL, [IQR]** | 6 [2;15] | 7 [3;21] | 3[1;9] |
| **Missing data** | 39 (35%) | 17 (25%) | 22 (49%) |

(continued)

| Variable | ALL N=112 | Other (autoimmune disease + idiopathic disease) N=67 | Neoplasia N=45 |
|---|---|---|---|
| **Median IL-6, pg/mL, [IQR]** | 46 [15;129] | 33 [10;128] | 75 [17;133] |
| **Missing data** | 6 (5%) | 2 (3%) | 4 (9%) |
| **Median IL-8, pg/mL, [IQR]** | 169 [47;673] | 192 [61;1063] | 161 [42;434] |
| **Missing data** | 5 (4%) | 3 (4%) | 2 (4%) |
| **Median IL-10, pg/mL, [IQR]** | 42 [16;173] | 26 [10;48] | 167 [51;997] |
| **Missing data** | 5 (4%) | 3 (4%) | 2 (4%) |
| **Median IFN-$\gamma$, pg/mL, [IQR]** | 160 [73;513] | 136 [73;516] | 202 [75;445] |
| **Missing data** | 18 (16%) | 6 (9%) | 12 (27%) |
| **Median TNF-$\alpha$, pg/mL, [IQR]** | 43 [25;73] | 55 [36;82] | 35 [15;44] |
| **Missing data** | 9 (8%) | 3 (4%) | 6 (13%) |
| **Median IL-18, pg/mL, [IQR]** | 574 [123;1859] | 256 [74;946] | 1045 [403;2494] |
| **Missing data** | 0 (0%) | 0 (0%) | 0 (0%) |
| **Median IP-10, pg/mL, [IQR]** | 10004 [2054;35450] | 6786 [1499;20975] | 21514 [3880;64888] |
| **Missing data** | 1 (1%) | 1 (1%) | 0 (0%) |
| **Median sIL2-R-$\alpha$, [IQR]** | 37 [6;92] | 13 [3;53] | 83 [35;189] |
| **Missing data** | 5 (4%) | 3 (4%) | 2 (4%) |
| **CD4/CD8 ratio, N (%)** | | | |
| **≤1** | 44 (54%) | 20 (43%) | 24 (71%) |
| **>1** | 37 (46%) | 27 (57%) | 10 (29%) |
| **Missing data** | 31 (28%) | 20 (30%) | 11 (24%) |

It was decided to look at the significance of values of markers, as compared to the median value of the markers within the cohort.

Univariate analysis made it possible to identify variables that are significantly associated with the presence of neoplasia.

**Table 3**. Results of the univariate analysis

| Variable | OR [95%CI] | p-value |
|---|---|---|
| Total lymphocytes > 730 /mm$^3$ | 0.52 [0.21 - 1.25] | 0.14 |
| Fibrinogen > 3 g/l | 1.41 [0.55 - 3.62] | 0.46 |

(continued)

| Variable | OR [95%CI] | p-value |
|---|---|---|
| AST >138 IU/l | 0.51 [0.23 - 1.15] | 0.10 |
| ALT >81 IU/l | 0.54 [0.25 - 1.19] | 0.13 |
| Hemoglobin > 9 g/l | 0.42 [0.19 - 0.92] | 0.03 |
| Platelets > 82 G/l | 0.40 [0.18 - 0.89] | 0.03 |
| Neutrophil > 2130 /mm$^3$ | 0.47 [0.20 - 1.12] | 0.09 |
| Ferritin > 7000 $\mu$g/L | 2.07 [0.93 - 4.62] | 0.08 |
| CD8/CD25+ > 73 c/mm$^3$ | 1.06 [0.17 - 6.54] | >0.9 |
| Age > 48y | 3.25 [1.46 - 7.21] | 0.004 |
| Female | 0.52 [0.23 - 1.14] | 0.10 |
| IL-1$\beta$ > 6 pg/mL | 0.84 [0.26 - 2.68] | 0.76 |
| IL-6 > 46 pg/mL | 2.30 [1.00 - 5.30] | 0.05 |
| IL-8 > 169 pg/mL | 0.84 [0.38 - 1.85] | 0.66 |
| IL-10 > 42 pg/mL | 5.36 [2.23 - 12.89] | 0.0003 |
| IFN-$_\gamma$ > 160 pg/mL | 1.03 [0.44 - 2.41] | > 0.9 |
| TNF-$\alpha$ > 43 pg/mL | 0.22 [0.09 - 0.51] | 0.0006 |
| IL-18 > 574 pg/mL | 5.26 [2.27 - 12.19] | 0.0002 |
| IP-10 > 10004 pg/mL | 2.02 [0.92 - 4.40] | 0.08 |
| sIL2-R-$\alpha$ > 37 pg/mL | 4.90 [2.13 - 11.27] | 0.0003 |
| CD4/CD8 ratio > 1 | 0.35 [0.14 - 0.91] | 0.03 |

The diagnostic score was constructed using multivariate logistic regression. A neoplasia-related risk test using the factors: age, IL10, TNF $\alpha$, IL18 and CD4/CD8 ratio (dichotomous 0/1 classification as compared to the median for each marker) was built

Predictive performance was assessed using an ROC curve and AUC for discrimination, and a Hosmer-Lemeshow test for calibration. Cross-validation (k-fold cross validation) was performed to correct for "over-optimism" (internal validation). The coefficients $\beta$ of the logistic regression are presented in Table 4. The coefficients ($\beta$ coefficients) were multiplied by 10 to obtain a score comprised between -28 and +37.

**Table 4**: coefficients (weight) assigned to each variable (marker)

| Variable | $\beta$ Coefficient | Score comprised between [-28 ; 37] |
|---|---|---|
| Age > 48 years | 1.1 | 11 |
| IL 10 > 42 pg/mL | 1.5 | 15 |
| TNF $\alpha$ > 43 pg/mL | -1.6 | -16 |
| IL 18 > 574 pg/mL | 1.1 | 11 |
| CD4/CD8 > 1 | -1.2 | -12 |

Example of how to calculate a patient's score based on the following characteristics:

- Age : 50 years
- Il 10 : 28.71 pg/mL
- TNF $\alpha$ : 81.22 pg/mL
- IL 18 : 842.71 pg/mL
- CD4/CD8 ratio > 1

Score :

$$11 + 0 + (-16) + 11 + (-12) = -6$$

The ROC curve represents the performance of a classification model for all classification thresholds. The curve plots sensitivity against 1 - specificity. The area under the ROC curve is 0.87 with a 95% confidence interval between 0.79 and 0.92, which means that the model's prediction quality is very good (figure 1).

Calibration was performed to determine the degree of correspondence between predicted and observed values (goodness of fit of predictive models to data). The Hosmer-Lemeshow test was therefore used to measure the goodness of fit of logistic models. Observed and predicted values are compared in subgroups of the population, fixed at 10. The p-value of the test was 0.30, which means that the predicted probabilities do not differ from the observed probabilities, and corresponds to excellent model calibration.

A k-fold cross-validation was carried out to avoid the overfitting. We set k to 10. The area under the ROC curve was 0.82 with a 95% confidence interval between 0.61 and 1.00.

In order to determine the most discriminating threshold (favoring specificity and sensitivity), sensitivity and specificity were calculated for different score thresholds. The threshold favoring specificity and sensitivity is 6, with a sensitivity of 0.8 and a specificity of 0.75 (PPV = 0.68 and NPV = 0.85).

Table 5. Logistic model

| Variable | Modality | OR | IC95% | p |
|---|---|---|---|---|
| Risk categories | Score < 6 | 1 | - | - |
| | Score $\geq$ 6 | 11.8 | [4.7 - 29.7] | <0.0001 |

According to the logistic model, we can observe that patients with a score $\geq$ 6 have a 12-fold higher risk of having a neoplasia associated with HLH than patients with a score < 6.

Summary

The cohort included 112 patients including 45 H-HLH and 67 AID or idiopathic HLHa. The median [IQR] age of patients in the cohort was 48 years [36-61] years old and 64 (57%) were men. From twenty-one potential predictors (including 9 HLH-related cytokines), eight were associated with H-HLH in univariate logistic regression: age > 48y (p<0.004), hemoglobin > 9g/l (p=0.03), platelets > 82G/l (p=0.03), !!-10 > 42 pg/mL (p=0.0003), TNF-$\alpha$>43 pg/mL (p=0.0006), IL-18>574 pg/mL (p=0.0002), lymphocytes CD4/CD8 >1 (p=0.03), and five were included in the risk score : age > 48y (OR [95%CI]: 3.0 [1.1 - 8.3], p=0.03), II-10 > 42 pg/mL (OR [95%CI]: 4.5 [1.4-14.6], p=0.01), TNF-$\alpha$>43 pg/mL (OR [95%CI]: 0.2 [0.07-0.57] ,p=0.003), IL-18>574 pg/mL (OR [95%CI]: 3.1[1.0-9.5], p=0.04) and lymphocytes CD4/CD8 >1 (OR [95%CI]: 0.3 [0.1-1.0], p=0.04). The score ranged from -28 and 37 with an attribution of points of 11 for age>48, 15 for II 10 > 42, -16 for TNF-$\alpha$ > 43, 11 for II 18 > 574 and -12 for CD4/CD8 > 1. The mean AUC in the cohort was 0.87 (95%CI, 0.79-0.92) and the AUC in the internal validation was 0.82 (95%CI, 0.61-1.00). The goodness-of-fit test showed a p-value of 0.30. The logistic model demonstrated that patients with a score $\geq$ 6 (sensitivity [95%CI]: 0.80 [0.65-0.90] and specificity [95%CI]: 0.75 [0.63-0.85] at the threshold of 6) the risk of having H-HLH increase by a factor of 12 compared to patients with a score < 6. (OR [95% CI] = 12 [4.7-29.7], p < 0.0001).

**Claims**

1. A method for determining whether an hemopathy is associated with Hemophagocytic Lymphohistiocytosis (HLH) in an adult subject with HLH, comprising

a) Obtaining the age in years of the subject
b) Obtaining the values of at least three biomarkers measured in the blood, plasma or serum of the subject, wherein the biomarkers are selected in the group consisting of hemoglobin, platelets, interleukin-1 beta(IL-1$\beta$), interleukin 6 (IL-6), interleukin 10 (IL-10), Tumor Necrosis Factor $\alpha$ (TNF-$\alpha$), interleukin 18 (IL-18), interferon gamma-induced protein 10 (IP-10), soluble Interleukin-2 receptor alpha chain (sIL2-R-$\alpha$, CD25), and CD4+/CD8+ ratio,
c) optionally obtaining values of other biomarkers
d) Combining values associated with the values obtained in a), b) and c) in a function, and obtaining an output
e) comparing the output to a predetermined cutoff value, and determining presence of an associated with HLH if the output is higher than the predetermined cutoff value.

2. The method of claim 1, wherein the value associated with each marker is obtained by

   a. comparing the values obtained in a), b) and c) to predetermined thresholds specific for each marker, and
   b. assigning the value 1 if the value is above the predetermined threshold for the marker, or the value 0 if the value is below or equal the predetermined threshold.

3. The method of claim 2, wherein the predetermined thresholds are

| Variable | Threshold |
|---|---|
| Age | 48 years |
| Hemoglobin | 9 g/l |
| Platelets | 82 G/l |
| IL-1$\beta$ | 6 pg/mL |
| IL-6 | 46 pg/mL |
| IL-10 | 42 pg/mL |
| TNF-$\alpha$ | 43 pg/mL |
| IL-18 | 574 pg/mL |
| IP-10 | 10004 pg/mL |
| sIL2-R-$\alpha$ | 37 pg/mL |
| CD4/CD8 ratio | 1 |

4. The method of claim 1, wherein the value associated with each marker is the value measured or obtained in a), b) and c).

5. The method of any one of claims 1 to 3, wherein the function is a logistic regression function.

6. The method of claim 4, wherein the function has been obtained by

   a. determining the presence of an hemopathy in a cohort of patients with HLH, wherein the values of circulating biochemical markers are known for the patients, and wherein the age is also known
   b. identifying by univariate logistic regression analysis, the circulating biochemical markers for which the values differ significantly between the group of patients with an hemopathy and the group of patients without presence of an hemopathy
   c. performing a multivariate logistic regression analysis to assess and ponder the independent discriminative value of the markers identified in step b) for the occurrence of hemopathy,
   d. thereby obtaining a function by combination of these identified independent factors.

7. The method of any one of claims 1 to 5, wherein at least four biomarkers are selected in b).

8. The method of any one of claims 1 to 7, wherein the biomarkers selected in b) are IL-10, TNF-$\alpha$, IL-18 and CD4/CD8 ratio.

9. The method of claim 1, wherein the biomarkers selected in b) are IL-10, TNF-$\alpha$, IL-18 and CD4/CD8 ratio, and wherein the function is
   $11 \times$ Age [0 if $\leq$48 years; 1 if >48 years] + $15 \times$ IL-10 [0 if $\leq$42; 1 if >42] - $16 \times$ TNF-$\alpha$ [0 if $\leq$43; 1 if > 43] + $11 \times$ IL-18 [0 if $\leq$ 574; 1 if > 574] - $12 \times$ CD4/CD8 ratio [0 if $\leq$ 1; 1 if > 1].

10. The method of claim 9, wherein the predetermined cutoff value is 6.

11. The method of any one of claims 1 to 10, wherein the patient has been diagnosed as having HLH by the H-test or the HLH-2004 test.

12. The method of any one of claims 1 to 10, which is implemented by computer.

13. A computer-implemented method for diagnosing the presence of an hemopathy associated with HLH in a subject, wherein the subject has HLH comprising

> a) Receiving inputs from a sender, wherein the inputs are the values of biomarkers measured in the blood, serum or plasma of the subject, as well as the information pertaining to the age of the subject
> b) Performing a method of any one of claims 1 to 12, to obtain an output for the combination of the values received in (a)
> c) optionally comparing the output to a reference value and determining the presence of the hemopathy associated with HLH
> d) Sending the output to the sender and/or the presence of the hemopathy associated with HLH, if determined
> e) Optionally storing the inputs and the output in a database.

14. A computer implemented method for obtaining information as to the presence of the hemopathy associated with HLH in a subject, wherein the subject has HLH comprising

> a) Sending inputs to a server, wherein the inputs are the values of the concentration of at least three measured biological markers selected from the group consisting of hemoglobin, platelets, interleukin-1 beta(IL-1$\beta$), interleukin 6 (IL-6), interleukin 10 (IL-10), Tumor Necrosis Factor $\alpha$ (TNF-$\alpha$), interleukin 18 (IL-18), interferon gamma-induced protein 10 (IP-10), soluble Interleukin-2 receptor alpha chain (sIL2-R-$\alpha$ , CD25), and CD4+/CD8+ ratio, and the age of the subject
> b) Having the remote server combine the values received in (a) in a function, described in any one of claims 1 to 12, and obtain an output [end result], optionally normalized
> c) optionally having the remote server compare the output to a reference value and determining the presence or absence of the hemopathy associated with HLH in the subject
> d) Optionally having the remote server store the inputs and the output in a database
> e) receiving the output from the server and/or the information pertaining to the presence or absence of the hemopathy associated with HLH (when determined).

15. A computer-implemented method for determining whether a patient with HLH has an hemopathy associated with HLH

> a) requiring a sender to identify himself to a server within a public or private network,
> b) requiring the sender to fill in information related to the patient, and assigning a specific identifier to the patient in a database, (this last step may be omitted if the patient has already been recorded in the database, as can be determined, using the patient information);
> c) receiving values of the concentration of at least three measured biological markers selected from the group consisting of hemoglobin, platelets, interleukin-1 beta(IL-1$\beta$), interleukin 6 (IL-6), interleukin 10 (IL-10), Tumor Necrosis Factor $\alpha$ (TNF-$\alpha$), interleukin 18 (IL-18), interferon gamma-induced protein 10 (IP-10), soluble Interleukin-2 receptor alpha chain (sIL2-R-$\alpha$ , CD25), and CD4+/CD8+ ratio, and the age (it may be calculated from the birth date), wherein the values are received in a secure manner, and storing the values in the database, associated with the specific identifier of the patient
> d) combining the values received or calculated in c) by use of a function as disclosed in any one of claims 1 to 12, obtaining a result, and optionally normalizing the result
> e) storing the result optionally normalized of d) in a database associated with the specific identifier and preferably the date and time of identification of the sender, thereby obtaining a database where the information related to the patient is present under the specific identifier,
> f) sending the nature of the presence of the hemopathy associated with HLH to the sender, wherein the patient has the hemopathy associated with HLH if the result is above a predetermined value and the patient has not the hemopathy associated with HLH if the result is below the or another predetermined value.

16. A device for determining whether a HLH patient has an hemopathy associated with HLH, comprising

> a. a keyboard for entering information
> b. software to present an information input interface
> c. a processor to calculate the values associated with the information entered by the user

d. a processor for calculating the output result according to a method according to one of claims 1 to 12
e. a monitor for displaying the output result, as well as the presence or absence of an hemopathy associated with HLH for the patient.

**Figure 1**

## EUROPEAN SEARCH REPORT

**Application Number**

EP 23 30 6315

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GAO WEI-BO ET AL: "A predictive model for identifying secondary underlying diseases of hemophagocytic lymphohistiocytosis", FRONTIERS IN IMMUNOLOGY, vol. 14, 28 April 2023 (2023-04-28), XP093120953, Lausanne, CH ISSN: 1664-3224, DOI: 10.3389/fimmu.2023.1143181 | 16 | INV. G01N33/68 G01N33/72 G16H50/20 |
| A | * the whole document, especially the abstract; page 2, right-column, 2nd par. – page 3, right column, 4th par.; section "prediction model" on page 5; figure 1 * | 1-7, 11-15 | |
| X | XIE M ET AL: "An effective diagnostic index for lymphoma-associated hemophagocytic syndrome", INTERNATIONAL JOURNAL OF MEDICINE, vol. 111, no. 8, 1 August 2018 (2018-08-01), pages 541-547, XP093121847, ISSN: 1460-2725, DOI: 10.1093/qjmed/hcy103 Retrieved from the Internet: URL:https://watermark.silverchair.com/hcy103.pdf?token=AQECAHi208BE49Ooan9kkhW_Ercy7Dm3ZL_9Cf3qfKAc485ysgAAA2cwggNjBgkqhkiG9w0BBwagggNUMIIDUAIBADCCA0kGCSqGSIb3DQEHATAeBglghkgBZQMEAS4wEQQMn6R4lZODO7yoQmcdAgEQgIIDGmsvxuDAcPf7Sj3cuHBL2MyGKkzVTb7pjlOH-B_iUiU4o0PO8Y9IFNLQ2y9CvVa3QqhdcV4d3QouRuNEzTy3wWvfPyyP-> | 16 | TECHNICAL FIELDS SEARCHED (IPC) G01N G16H |
| A | * abstract; page 542, left-column, section "patients and data collection" – page 545, left-column, 1st par. * | 1-7, 11-15 | |

-/--

The present search report has been drawn up for all claims

| Place of search | Date of completion of the search | Examiner |
|---|---|---|
| Munich | 22 January 2024 | Lindberg, Pia |

EPO FORM 1503 03.82 (P04C01)

Europäisches
Patentamt

European
Patent Office

Office européen
des brevets

# EUROPEAN SEARCH REPORT

**Application Number**

EP 23 30 6315

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MARUOKA HAYATO ET AL: "IP-10/CXCL10 and MIG/CXCL9 as novel markers for the diagnosis of lymphoma-associated hemophagocytic syndrome", ANNALS OF HEMATOLOGY, vol. 93, no. 3, 25 August 2013 (2013-08-25), pages 393-401, XP093121295, DE ISSN: 0939-5555, DOI: 10.1007/s00277-013-1878-y Retrieved from the Internet: URL:http://link.springer.com/content/pdf/10.1007/s00277-013-1878-y.pdf> | 16 | |
| A | * abstract; page 394, right-column, 2nd full par. - page 396, right-column, 2nd par. * | 1-7, 11-15 | |
| X | ZOREF-LORENZ ADI ET AL: "An improved index for diagnosis and mortality prediction in malignancy-associated hemophagocytic lymphohistiocytosis", BLOOD, AMERICAN SOCIETY OF HEMATOLOGY, US, vol. 139, no. 7, 17 November 2021 (2021-11-17), pages 1098-1110, XP086965116, ISSN: 0006-4971, DOI: 10.1182/BLOOD.2021012764 [retrieved on 2021-11-17] | 16 | **TECHNICAL FIELDS SEARCHED (IPC)** |
| A | * abstract: page 1102, left-column, section "statistical analysis" - page 1104, right-column, 2nd par.; figure 2 * | 1-7, 11-15 | |

-/--

The present search report has been drawn up for all claims

| Place of search | Date of completion of the search | Examiner |
|---|---|---|
| Munich | 22 January 2024 | Lindberg, Pia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 23 30 6315

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NAVAL DAVER ET AL: "A consensus review on malignancy-associated hemophagocytic lymphohistiocytosis in adults", CANCER, AMERICAN CANCER SOCIETY , PHILADELPHIA , PA, US, vol. 123, no. 17, 16 June 2017 (2017-06-16), pages 3229-3240, XP071127452, ISSN: 0008-543X, DOI: 10.1002/CNCR.30826 | 16 | |
| A | * abstract; page 3232, left-column, section "secondary M-HLH" - page 3236, left-column, 1st par; figure 2 * | 1-7, 11-15 | |
| X | REN QUANGUANG ET AL: "Platelet-derived alpha-granules are associated with inflammation in patients with NK/T-cell lymphoma-associated hemophagocytic syndrome", CYTOKINE, ACADEMIC PRESS LTD, PHILADELPHIA, PA, US, vol. 126, 15 November 2019 (2019-11-15), XP085973015, ISSN: 1043-4666, DOI: 10.1016/J.CYTO.2019.154878 [retrieved on 2019-11-15] | 16 | |
| A | * abstract; sections 2.1-3.2 on pages 2-3; page 7, section "conclusions" * | 1-7, 11-15 | |

**TECHNICAL FIELDS SEARCHED (IPC)**

The present search report has been drawn up for all claims

| Place of search | Date of completion of the search | Examiner |
|---|---|---|
| Munich | 22 January 2024 | Lindberg, Pia |

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

**see sheet B**

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

**1-7, 11-16(all partially)**

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**

**SHEET B**

Application Number

**EP 23 30 6315**

The Search Division considers that the present European patent application does not comply with the requirements of unity of invention and relates to several inventions or groups of inventions, namely:

```
1. claims: 1-7, 11-16(all partially)

        Methods for determining whether an hemopathy is associated
        with Hemophagocytic Lymphohistiocytosis (HLH) in an adult
        subject with HLH, wherein the at least three biomarkers used
        in the method are hemoglobin, platelets and
        interleukin-1beta (IL-1?), and a corresponding device.
                          ---


2. claims: 8-10(completely); 1-7, 11-16(partially)

        Methods for determining whether an hemopathy is associated
        with Hemophagocytic Lymphohistiocytosis (HLH) in an adult
        subject with HLH, wherein the biomarkers are IL-10, TNF-?,
        IL-18 and CD4/CD8 ratio and a corresponding device.
                          ---


3. claims: 1-7, 11-16(all partially)

        Methods for determining whether an hemopathy is associated
        with Hemophagocytic Lymphohistiocytosis (HLH) in an adult
        subject with HLH, wherein the at least three biomarkers used
        in the method are hemoglobin, platelets and interleukin 6
        (IL-6), and a corresponding device.
                          ---


4. claims: 1-7, 11-16(all partially)

        Methods for determining whether an hemopathy is associated
        with Hemophagocytic Lymphohistiocytosis (HLH) in an adult
        subject with HLH, wherein the at least three biomarkers used
        in the method are one of the possible combination of at
        least three biomarkers selected from the list of biomarkers
        as in claim 1, and a corresponding device.
        It is noted that a high number of possible at least 3
        biomarker combinations (120) are possible from the 10
        different biomarkers as in the list of claim 1. Therefore
        the number of inventions in the search report is set
        exemplary to only 4.
                          ---
```

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HENTER et al.** *Pediatr Blood Cancer.*, February 2007, vol. 48 (2), 124-31
- **FARDET et al.** *Arthritis Rheumatol.*, September 2014, vol. 66 (9), 2613-20
- **WANG et al.** *Oncotarget.*, 14 July 2017, vol. 8 (35), 59977-59985
- **ZHOU et al.** *J Clin Immunol.*, July 2022, vol. 42 (5), 1000-1008
- **SHIGA et al.** *Front Immunol.*, 08 October 2021, vol. 12, 750114
- **GAO et al.** *Front Immunol.*, 28 April 2023, vol. 14, 1143181
- **KEENAN et al.** *Front Immunol.*, 16 February 2021, vol. 12, 614704
- **DAS et al.** *Blood.*, 31 March 2016, vol. 127 (13), 1666-75